# EUROPÄISCHE PATENTSCHRIFT

(11) **EP 3 655 960 B1**
(45) Veröffentlichungstag und Bekanntmachung des Hinweises auf die Patenterteilung: **29.09.2021**
(21) Anmeldenummer: 18765545.1
(22) Anmeldetag: 18.07.2018
(51) Int. Cl.: G16C 10/00

(54) **SENSOREINRICHTUNG ZUR UNTERSTÜTZUNG EINES HELFERS BEI EINER HERZ-LUNGEN-WIEDERBELEBUNG**
SENSOR DEVICE FOR ASSISTING AN AIDER WITH A CARDIOPULMONARY RESUSCITATION
DISPOSITIF CAPTEUR POUR ASSISTER UN SECOURISTE LORS D'UNE RÉANIMATION CARDIO-PULMONAIRE

(30) Priorität: 20.07.2017 DE 102017116361
(43) Veröffentlichungstag der Anmeldung: 27.05.2020
(73) Patentinhaber: Müller, Michael, 79117 Freiburg (DE); Roth, Matthias, 79111 Freiburg (DE); Schorling, Per, 5700 Svendborg (DK)
(72) Erfinder: Müller, Michael, 79117 Freiburg (DE); Roth, Matthias, 79111 Freiburg (DE); Schorling, Per, 5700 Svendborg (DK)
(74) Vertreter: Kaufmann, Sigfrid
(86) Internationale Anmeldenummer: PCT/DE2018/100656
(87) Internationale Veröffentlichungsnummer: WO 2019/015727

(56) Entgegenhaltungen:
- WO-A1-2013/093757
- WO-A1-2013/128369
- WO-A1-2015/066430
- WO-A1-2015/189275
- WO-A2-2015/048323
- CH-A2- 710 687
- KR-A- 20130 122 822
- US-A1- 2008 171 311

## Beschreibung

Die Erfindung betrifft eine Sensoreinrichtung zur Erfassung von Daten bei der Durchführung von Erste-Hilfe-Maßnahmen zur Reanimation eines von einem Kreislaufstillstand betroffenen Patienten. Die Sensoreinrichtung ist in Verbindung mit einem Anzeige- und Feedbackgerät, das eingerichtet ist, auf der Grundlage des Unterschieds zwischen der durchgeführten Herz-Lungen-Wiederbelebung und der empfohlenen Herz-Lungen-Wiederbelebung anzuleiten, oder in Kombination mit einem Defibrillator anwendbar zur Wiederbelebung eines Patienten mit Kreislaufstillstand, insbesondere durch einen Laienhelfer in einer Notfallsituation oder zur Übung einer Reanimation.

Der plötzliche Herztod ist eine der häufigsten Todesursachen. Trotz erheblicher Bemühungen in den letzten Dekaden sowie der Etablierung internationaler Leitlinien für die Behandlung des Herzkreislaufstillstands liegt die Überlebensrate nur bei etwa 10%. Zum Beispiel erleiden jährlich in Deutschland 50.000 Menschen einen Kreislaufstillstand, von denen die meisten sterben oder nur mit bleibenden neurologischen Schäden überleben.

Überleben nach dem plötzlichen Herztod ist nur möglich, wenn innerhalb der ersten Minuten und noch vor dem Eintreffen des Rettungsdienstes mit einer Herzdruckmassage begonnen wird und diese entsprechend der jeweils aktuellen Empfehlungen der Leitlinien erfolgt. Momentan empfehlen diese eine Eindrucktiefe von 5-6 cm, eine Kompressions-Frequenz von etwa 100 pro Minute sowie eine komplette Entlastung nach jeder Thoraxkompression. Die Unterbrechungen der Herzdruckmassage - um beispielsweise zu beatmen oder einen Defibrillationsschock auszulösen - sollen so kurz wie möglich sein, damit das Gehirn möglichst ununterbrochen durchblutet wird und keine irreversiblen Schäden auftreten.

Beispielsweise leiten in Deutschland nur in etwa 40% der Notfälle Laienhelfer die wichtigen Maßnahmen der Wiederbelebung (vor allem Herzdruckmassage) ein. Die Qualität der Herzdruckmassage nach Schulungsmaßnahmen zu deren Grundlagen ist gut, nimmt aber innerhalb von sechs Monaten wieder erheblich ab. Zudem sinkt auch während einer Wiederbelebung die Qualität der Herzdruckmassage bereits nach zwei Minuten erheblich ab.

Die Überlebenswahrscheinlichkeit eines Patienten mit Kreislaufstillstand hängt wesentlich von den Maßnahmen des Laienhelfers ab. Dieser ist jedoch mit der Situation häufig überfordert und selten ausreichend trainiert.

Aus EP 1 128 795 B1 ist ein System zum Messen und Veranlassen von Brustkompressionen bekannt. Es umfasst einen mobilen CPR-Kompressionsmonitor (CPR - Cardiopulmonale Reanimation, Herz-Lungen-Wiederbelebung) zur Überwachung der Thoraxkompressionen zur Reanimation einer von einem Kreislaufstillstand betroffenen Person. Das Gerät wird auf der Hand des Helfers oder auf dem Patienten abgelegt und umfasst Beschleunigungssensoren sowie eine Schnittstelle zur Datenübertragung. An diese Schnittstelle wird über ein Kabel eine im CPR-Kompressionsmonitor integrierte oder eine eigenständige Auswerteeinheit mit Bildschirm angeschlossen. Dieses System ist für geschultes und erfahrenes medizinisches Personal konzipiert.

WO 2006/104977 A2, EP 2 255 845 A1 und DE 60 2004 002 147 T2 offenbaren ein vielschichtiges professionelles medizinisches System zur Unterstützung eines Ersthelfers, wobei dieser in der Reanimation geschult sein muss. Das System umfasst unter anderem einen Defibrillator und ein mobiles Anzeige- und Steuergerät.

Desgleichen beschreibt EP 1 858 472 B1 ein zwar mobiles, doch sehr komplexes medizinisches System zur Unterstützung eines Helfers bei der Reanimation, das auch einen Defibrillator umfasst. Es ist vorgesehen, das Gerät an wenigen zentralen Orten mit hohem Menschenaufkommen zu stationieren, damit Ersthelfer einen schnellen Zugriff darauf erhalten. Allerdings ist auch dieses Gerät nur von geschulten Helfern einigermaßen sinnvoll einsetzbar.

Verfahren und Vorrichtungen zur exakten Bestimmung der Eindrücktiefe bei Brustkorbkompressionen sind in WO 2004/ 037 154 A2 und DE 11 2010 000 978 T5 gezeigt.

Wesentliche Nachteile bei den aus dem Stand der Technik bekannten Lösungen bestehen darin, dass sie vergleichsweise groß und schwer sind. Auch sind entsprechende Hilfsmaßnahmen bei deren Anwendung zeitaufwendig, wobei ein (Erst-)Helfer häufig überfordert ist, insbesondere wenn er vor Eintritt des Notfalls nicht ausreichend an dem jeweiligen Gerät trainiert wurde.

WO 2015/110118 beschreibt ein System zur Unterstützung eines Helfers bei der Reanimation einer Person mit Kreislaufstillstand, das ein Heftpflaster mit einem darauf befestigten Lagesensor umfasst, wobei der Lagesensor die Eindrucktiefe und die Massagefrequenz an ein mit dem Lagesensor in Kommunikationsverbindung stehendes Smartphone übermittelt.

WO 2015/066430 A1 offenbart eine Vorrichtung zur Unterstützung des medizinischen Personals bei Behandlung von Patienten mit Herzinsuffizienz, die eine auf den Körper des Patienten adhäsiv aufbringbare Sensoreinrichtung umfasst. Die elektronischen Bauteile sind innerhalb einer wasserdichten Einhausung der Sensoreinrichtung angeordnet.

CH 710 687 A2 beschreibt eine Vorrichtung zur Unterstützung der Reanimation einer Person mit Kreislaufstillstand in Form eines Handschuhs, den der Helfer zur Durchführung der Reanimation überstreift. Die elektronischen Bauelemente sind im Handschuh integriert.

Der Erfindung liegt die Aufgabe zugrunde, eine Sensoreinrichtung zur Erfassung von Daten bei der Durchführung von Ersthilfsmaßnahmen zur Reanimation einer von einem Kreislaufstillstand betroffenen Person, nachfolgend Patient genannt, bereitzustellen, die es auch einem ungeschulten Laienhelfer ermöglicht, unverzüglich erste lebensrettende Maßnahmen durchzuführen, wobei die Sensoreinrichtung so klein und kompakt sein soll, dass sie stets mitgeführt werden kann, und wobei die Sensoreinrichtung in ihrer Handhabung absolut intuitiv sein soll, sodass ihre Anwendung zur Reanimation auch in Stresssituationen und ohne medizinische Vorkenntnisse ermöglicht ist.

Die Aufgabe wird gemäß der Erfindung durch eine Sensoreinrichtung mit den kennzeichnenden Merkmalen nach dem Patentanspruch 1 gelöst; vorteilhafte Weiterbildungen der Erfindung sind in den Unteransprüchen 2 bis 13 beschrieben. Eine zusätzliche Verwendung der Erfindung ist in Anspruch 14 aufgezeigt.

Gemäß der Erfindung wird eine Sensoreinrichtung zur Erfassung der bei der Herzdruckmassage relevanten Daten und dadurch zur Unterstützung eines Reanimators, d. h. Helfers, insbesondere eines in der Reanimation unausgebildeten Laienhelfers, bei der Reanimation des Patienten mit einem Kreislaufstillstand bereitgestellt.

Die Sensoreinrichtung umfasst einen weitestgehend steifen, vorzugsweise zumindest bereichsweise starren, flach ausgestalteten Gehäusekörper, der wasserdicht ausgebildet ist. Der Gehäusekörper besitzt z. B. die Form einer Linse oder einer Muschel. Insbesondere kann der Gehäusekörper - zumindest bereichsweise - eine Elastizität aufweisen, die eine Anpassung desselben an die Form der Hand des Reanimators bzw. des Brustkorbes des Patienten bei Ausübung der Herzdruckmassage, z. B. bei einer einwirkenden Kraft von etwa 800 N, ermöglicht. Somit kann der Druck vergleichmäßigt und Hautschäden vorgebeugt werden.

Innerhalb des Gehäusekörpers sind wenigstens ein Bewegungssensor, ein Speicher für elektrische Energie, nachfolgend Stromspeicher genannt, ein Prozessor und eine Schnittstelle zum Senden und/oder Empfangen von Daten und/oder Befehlen angeordnet.

Die Schnittstelle kann drahtlos ausgeführt sein, wobei sie vorzugsweise eingerichtet ist, elektromagnetische Wellen zu senden bzw. zu empfangen, z. B. nach einem Bluetooth-, einem NFC- oder einem WLAN-Standard,
Die Sensoreinrichtung weist außerdem wenigstens ein Befestigungsmittel zur Befestigung sowie eine Positionierungshilfsvorrichtung zur exakten Positionierung des Gehäusekörpers auf dem Brustkorb des Patienten auf. Die am Gehäusekörper angebrachte bzw. in diesen integrierte Positionierungshilfsvorrichtung ist in Form einer Maßverkörperung, z. B. eines Bandes, einer Schnur oder eines Stabes einer vorgegebenen Länge, ausgebildet, die aus dem Gehäuse herausklapp- oder herausziehbar ist. Vorzugsweise ist die Positionierungshilfsvorrichtung eine herausziehbare, z. B. abrollbare, flexible Maßverkörperung, deren maximale Länge 8 cm, vorzugsweise 5 cm, nicht übersteigt.

Auf der Außenseite des Gehäuses weist dasselbe zumindest auf einem Bereich der Oberfläche, der - bei bestimmungsgemäßen Gebrauch der Sensorvorrichtung während der Reanimation - mit z. B. der vermittels der Sensoreinrichtung auf den Brustkorb des Patienten drückenden Hand des Reanimators in Kontakt tritt, eine Klebefläche in Form einer adhäsiven Beschichtung auf. Somit wird durch die mittelbare Verbindung der Hand mit dem Brustkorb - in Kombination mit der Form des Gehäuses - eine Sogwirkung während des zweiten Bewegungsabschnittes der Herzdruckmassage erzeugt, wodurch eine aktive Entlastung des Brustkorbes ermöglicht ist.

Die Sensoreinrichtung kann als ein flacher Anhänger ausgebildet sein, vorzugsweise in Form eines an einem z. B. Schlüsselbund befestigbaren Schlüsselanhängers, dessen Abmessungen im Transportzustand kleiner als 10 cm x 10 cm x 2 cm sind.

Der Prozessor ist derart ausgestaltet, dass die mittels des wenigstens einen Sensors erfassten Daten auswertbar und in einer von z. B. einem über die Schnittstelle mit der Sensoreinrichtung kommunizierenden mobilen Endgerät darstellbaren bzw. weiterverarbeitbaren Form aufbereitbar sind. Das mit der Sensoreinrichtung kommunizierende mobile Endgerät kann jede Art von eigenständigem, tragbaren Monitor, Industriedisplay oder ein Klein- oder Kleinstcomputer, zum Beispiel eine Smartwatch, ein Smartphone, ein Phablet, ein Tablet oder ein Netbook, sein.

Der Vorteil der Sensoreinrichtung liegt darin, dass sie aufgrund ihrer Ausgestaltung, insbesondere ihren geringen Abmessungen, von praktisch jedem Hilfswilligen und jederzeit mitgeführt werden kann. So ist sie, bei Vorhandensein eines kompatiblen mobilen Endgerätes, sofort einsetzbar, d. h. langwierige Vorbereitungen oder das Warten auf medizinisch ausgebildetes Hilfspersonal können entfallen.

Zusätzlich kann die Sensoreinrichtung eine Aktivierungsvorrichtung, z. B. in Form eines Schalters oder einer Lasche mittels derer die elektronischen bzw. elektrischen Komponenten im Gehäuse, z. B. der Prozessor, mit dem Speicher für elektrische Energie verbindbar sind, besitzen.

Das Befestigungsmittel der Sensoreinrichtung ist vorzugsweise als medizinisches Klebeband, z. B. als Heftpflaster, ausgebildet. Hierbei kann - z. B. nach Art von Flügeln - an zwei gegenüberliegenden Längsseiten des Gehäusekörpers der Sensoreinrichtung je ein Heftpflaster befestigt oder herausziehbar sein, die im Lagerungs- bzw. Transportzustand der Sensoreinrichtung beispielsweise auf den Gehäusekörper der Sensoreinrichtung umgeklappt bzw. im Gehäusekörper verstaut sind.

Gemäß einer alternativen Ausgestaltung kann das Befestigungsmittel als Klettverschluss bzw. als Klettband mit Widerhaken oder Schlaufen ausgebildet sein.

Der Stromspeicher kann eine Einweg-Batterie oder eine wiederaufladbare Batteriezelle sein, wobei das Laden des Stromspeichers induktiv erfolgen kann, d. h. die Sensoreinrichtung umfasst in diesem Fall zusätzlich eine induktive Lade-Schnittstelle zur Ankopplung an eine induktive Ladevorrichtung.

Vor einer Verwendung der Sensoreinrichtung kann ihre Aktivierung mittels Betätigens der Aktivierungsvorrichtung erforderlich sein, indem z. B. ein Teil des Gehäusekörpers oder eine Lasche an einer Sollbruchstelle umgeknickt oder abgebrochen, eine z. B. als Isolator ausgebildete Lasche aus dem Gehäusekörper herausgezogen oder indem z. B. das als Heftpflaster ausgebildete Befestigungsmittel aus dem Gehäusekörper herausgezogen wird. Es kann auch vorgesehen sein, dass die Aktivierungsvorrichtung mit der Positionierungshilfsvorrichtung gekoppelt ist, sodass ein Betätigen der Positionierungshilfsvorrichtung für eine exakte Positionierung der Sensoreinrichtung auf dem Brustkorb des Patienten die Stromversorgung herstellt.

Alternativ oder zusätzlich kann die Aktivierungsvorrichtung einen innerhalb des Gehäuses angeordneten Schalter, der z. B. einen Druck- bzw. Kraftsensor umfasst, aufweisen, der die Sensoreinrichtung aktiviert, sobald der Helfer vermittels der Sensoreinrichtung auf den Brustkorb des Patienten presst. Insbesondere kann dieser Schalter ausgebildet sein, erst oberhalb einer vorgegebenen Presskraft, z. B. 800 N, auszulösen.

Gemäß einer Ausführungsform ist der Bewegungssensor ein Beschleunigungssensor, vorzugsweise ein Drei-Achs-Beschleunigungssensor, wobei aus den von dem Sensor erfassten Beschleunigungswerten sowohl die Eindrucktiefe wie auch die Kompressionsfrequenz errechnet werden können. Auch kann vorgesehen sein, dass die Sensoreinrichtung zwei, insbesondere redundante, Bewegungssensoren umfasst.

Die Sensoreinrichtung kann weiterhin zusätzliche Sensoren, z. B. einen Kraftsensor, umfassen.

Es kann vorgesehen sein, dass die Maßverkörperung Markierungspositionen - z. B. ausgebildet als Einrastpunkte, die jeweils eine vorgegebene Ausziehlänge der Maßverkörperung beim Herausziehen aus dem Gehäusekörper definieren - für Patienten unterschiedlicher Körpergröße aufweist, z. B. in Form einer Markierung für Kinder, für Jugendliche und für erwachsene Männer oder Frauen. Somit ist mittels Anlegens der Maßverkörperung an das Brustbein eine exakte Platzierung der Sensoreinrichtung auf dem Brustbein des Patienten ermöglicht.

Insbesondere kann die Erfindung derart ausgestaltet sein, dass die Maßverkörperung aus einem flexiblen oder starren faserverstärktem Kunststoff, z. B. carbonfaserverstärkter Kunststoff (CFK) oder aus Aramid, besteht, wobei dessen temperaturabhängiger Längenausdehnungskoeffizient im Wesentlichen Null betragen kann.

Gemäß einer Ausführungsform kann die Maßverkörperung elektrisch leitend sein oder einen elektrischen Leiter umfassen, wobei sie mit der drahtlosen Schnittstelle verbunden ist. Somit ist die Maßverkörperung in vorteilhafter Weise als Antenne für das Senden und/oder Empfangen von Daten und/oder Befehlen ausgebildet. Alternativ oder zusätzlich kann vorgesehen sein, dass die Maßverkörperung eine einseitig, zumindest partiell aufgebrachte Klebeschicht aufweist, sodass bei Betätigen der Positionierungshilfsvorrichtung die Maßverkörperung auf dem Brustkorb des Patienten mittels der Klebeschicht fixierbar ist.

Die Sensoreinrichtung kann weiter derart ausgebildet sein, dass sie einen induktiven Sensor zur Detektion von durch den Brustkorb des Patienten fließenden elektrischen Strömen umfasst. Mittels dieses Sensors kann somit ein Defibrillationsschock identifiziert werden.

Gemäß einer Ausführungsform kann die auf dem im bestimmungsgemäßen Gebrauch der Sensoreinrichtung mit der Hand des Reanimators in Kontakt tretende Oberflächenbereich des Gehäusekörpers aufgebrachte adhäsive Beschichtung ein Gel oder Silikon sein. Hierbei kann vorgesehen sein, dass die adhäsive Beschichtung von einer Schutzabdeckung geschützt ist, die z. B. bei oder zur Aktivierung der Sensoreinrichtung entfernt wird.

Weiterhin kann die Sensoreinrichtung eine akustische und/oder eine haptische Feedbackeinrichtung aufweisen, die beispielsweise mittels Vibration eine Abweichung und/oder eine Übereinstimmung zwischen der durchgeführten Herz-Lungen-Reanimation und der empfohlenen Herz-Lungen-Reanimation signalisiert. Die Feedbackeinrichtung kann von einem externen, über die Schnittstelle an die Sensoreinrichtung anzuschließenden, mobilen Endgerät angesteuert werden. Alternativ oder zusätzlich kann dies auch von dem Prozessor der Sensoreinrichtung erfolgen.

Zur Verwendung der Sensoreinrichtung wird diese mittels des Befestigungsmittels auf dem Brustbein eines Patienten oder einer Übungspuppe fixiert, sodass der Reanimator über die Sensoreinrichtung auf den Brustkorb drückt. Somit wird die Sensoreinrichtung bei Durchführung einer Herzdruckmassage, d. h. Thoraxkompressionen, entsprechend der Bewegungen des Brustbeins mitbewegt. Bei der Durchführung von Thoraxkompressionen können die damit einhergehenden Kompressionsbewegungen mittels der Sensoren erfasst werden.

Die Sensoreinrichtung wird mittels ihrer Schnittstelle mit einem mobilen Endgerät in Kommunikationsverbindung gebracht. Das mobile Endgerät kann insbesondere derart ausgebildet sein, dass von ihm die mittels der Sensoreinrichtung ermittelten Daten zur Bestimmung der Eindrucktiefe (um die das Brustbein bei der Thoraxkompression eingedrückt wird) und/oder die Frequenz der Thoraxkompressionen empfangen werden und basierend darauf die Anleitung des Helfers erfolgt.

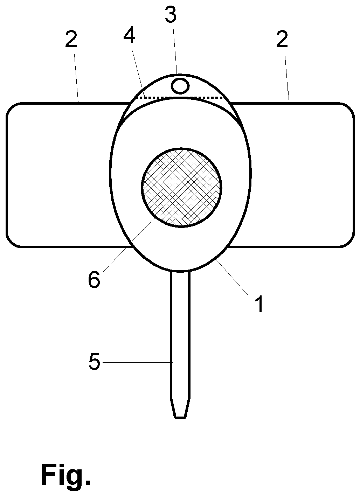

Die Erfindung wird nachfolgend anhand eines Ausführungsbeispiels näher erläutert, wobei gleiche oder ähnliche Merkmale mit gleichen Bezugszeichen versehen sind. Dabei zeigt die Figur eine schematische Draufsicht auf die Sensoreinrichtung.

Die als Schlüsselanhänger ausgebildete Sensoreinrichtung umfasst den steifen, flachen Gehäusekörper 1 und das Befestigungsmittel 2 in Form von zwei seitlich am Gehäusekörper 1 angebrachten, flexiblen Pflasterstreifen, die im Transportzustand des Schlüsselanhängers auf den Gehäusekörper 1 geklappt sind. Die Lasche 3 dient zur Befestigung am Schlüsselring. Auf den Gehäusekörper 1 ist die adhäsive Beschichtung 6 aufgebracht.

Für eine Aktivierung der Sensoreinrichtung ist die Lasche 3 an der Sollbruchstelle 4 abzuknicken. Dadurch kann über eine Bluetooth-Schnittstelle (nicht dargestellt) der Sensoreinrichtung eine drahtlose Verbindung mit einem Smartphone zum Empfang der mit der Sensoreinrichtung erfassten Daten hergestellt werden.

Die Positionierungshilfsvorrichtung 5 wird, nach ihrem Herausziehen aus dem Gehäusekörper 1, mit ihrer Spitze am unteren Teil des Brustbeines angelegt, wodurch die Sensoreinrichtung genau an der medizinisch korrekten Position auf dem Brustkorb des Patienten zu liegen kommt.

### Bezugszeichenliste

- 1: Gehäusekörper
- 2: Befestigungsmittel
- 3: Lasche
- 4: Sollbruchstelle
- 5: Positionierungshilfsvorrichtung
- 6: adhäsive Beschichtung

## Patentansprüche

1. Sensoreinrichtung zur Unterstützung eines Helfers bei einer Herz-Lungen-Wiederbelebung eines von einem Kreislaufstillstand betroffenen Patienten, umfassend Befestigungsmittel (2) zur Befestigung der Sensoreinrichtung auf dem Brustkorb des Patienten, einen Bewegungssensor und eine Schnittstelle zum Senden und/oder Empfangen von Daten und/oder Befehlen,
wobei die Sensoreinrichtung einen weitestgehend flachen, wasserdichten Gehäusekörper (1) aufweist, in welchem der Bewegungssensor, ein Speicher für elektrische Energie, ein Prozessor und die Schnittstelle eingeschlossen sind, und wobei der Gehäusekörper (1) eine Oberseite, die im bestimmungsgemäßen Gebrauch der Sensoreinrichtung eine Hand des Helfers kontaktiert, und eine Unterseite, die im bestimmungsgemäßen Gebrauch der Sensoreinrichtung den Brustkorb des Patienten kontaktiert, aufweist,
**dadurch gekennzeichnet, dass**
die Sensoreinrichtung weiterhin aufweist:
- eine Positionierungshilfsvorrichtung (5) zur exakten Positionierung der Sensoreinrichtung auf dem Brustkorb des Patienten, wobei die Positionierungshilfsvorrichtung (5) eine aus dem Gehäusekörper (1) herausklappoder herausziehbare Maßverkörperung ist; und
- eine adhäsive Beschichtung (6) zumindest auf einem Oberflächenbereich der Oberseite des Gehäusekörpers (1).

2. Sensoreinrichtung nach Anspruch 1, **dadurch gekennzeichnet, dass** sie eine Aktivierungsvorrichtung zum Verbinden zumindest des Prozessors mit dem Speicher für elektrische Energie aufweist.

3. Sensoreinrichtung nach Anspruch 2, **dadurch gekennzeichnet, dass** die Aktivierungsvorrichtung mittels Betätigung einer Sollbruchstelle (4) oder Entfernens einer Lasche aus dem Gehäuse (1) schaltbar ist.

4. Sensoreinrichtung nach Anspruch 2, **dadurch gekennzeichnet, dass** die Aktivierungsvorrichtung ein innerhalb des Gehäuses (1) angeordneter, druck- oder bewegungsempfindlicher Aktivierungsschalter ist.

5. Sensoreinrichtung nach Anspruch 2, **dadurch gekennzeichnet, dass** die Aktivierungsvorrichtung ein Bestandteil der Positionierungshilfsvorrichtung (5) oder mit derselben gekoppelt ist.

6. Sensoreinrichtung nach einem der vorstehenden Ansprüche, **dadurch gekennzeichnet, dass** die Schnittstelle drahtlos ist.

7. Sensoreinrichtung nach einem der vorstehenden Ansprüche, **dadurch gekennzeichnet, dass** der Speicher für elektrische Energie wiederaufladbar ist, wobei die Sensoreinrichtung eine induktive Lade-Schnittstelle für eine induktive Ladevorrichtung aufweist.

8. Sensoreinrichtung nach einem der vorstehenden Ansprüche, **dadurch gekennzeichnet, dass** der Bewegungssensor ein Beschleunigungssensor ist.

9. Sensoreinrichtung nach einem der vorstehenden Ansprüche, **dadurch gekennzeichnet, dass** sie auf dem mit dem Brustkorb in Kontakt tretenden Bereich der Gehäusewand einen Klettverschluss aufweist.

10. Sensoreinrichtung nach einem der vorstehenden Ansprüche, **dadurch gekennzeichnet, dass** die Maßverkörperung aus faserverstärktem Kunststoff besteht.

11. Sensoreinrichtung nach einem der vorstehenden Ansprüche, **dadurch gekennzeichnet, dass** sie einen induktiven Sensor zur Detektion von durch den Brustkorb des Patienten fließenden elektrischen Strömen umfasst.

12. Sensoreinrichtung nach einem der vorstehenden Ansprüche, **dadurch gekennzeichnet, dass** das Befestigungsmittel (2) in Form von zwei, jeweils seitlich und einander gegenüberliegend am Gehäusekörper (1) angeordneten flexiblen Klebestreifen, die für eine Befestigung der Sensoreinrichtung aus dem Gehäusekörper (1) herausziehbar oder von dem Gehäusekörper (1) wegklappbar sind, ausgebildet ist.

13. Sensoreinrichtung nach einem der vorstehenden Ansprüche, **dadurch gekennzeichnet, dass** die adhäsive Beschichtung (6) ein Gel oder Silikon ist.

14. Verwendung der Sensoreinrichtung nach einem der vorstehenden Ansprüche zur Analyse des Bewegungsablaufes eines Lebewesens oder einer Maschine.

## Claims

1. Sensor device for assisting an aider with a cardiopulmonary resuscitation of a patient affected by a cardiac arrest, comprising fastening means (2) for fastening the sensor device to the chest of a patient, a motion sensor and an interface for transmitting and/or receiving data and/or commands,
whereby the sensor device has a largely flat watertight housing body (1) which encloses the motion sensor, a store for electrical energy, a processor and the interface, and whereby the housing body (1) has an upper side, which contacts a hand of the aider during the intended use of the sensor device, and a lower side which contacts the chest of the patient during the intended use of the sensor device,
**characterised in that**
the sensor device also has:
- a positioning aid apparatus (5) for exact positioning of the sensor device on the chest of the patient, whereby the positioning aid apparatus (5) is a measuring standard which can be folded out or pulled out of the housing body (1); and
- an adhesive coating (6) at least on a surface region of the upper side of the housing body (1).

2. Sensor device according to claim 1, **characterised in that** it has an activation apparatus for connecting at least the processor to the store for electrical energy.

3. Sensor device according to claim 2, **characterised in that** the activation apparatus is switchable by means of actuation of a predetermined breaking point (4) or removal of a tab from the housing (1).

4. Sensor device according to claim 2, **characterised in that** the activation apparatus is a pressure-sensitive or motion-sensitive activation switch which is arranged inside the housing (1).

5. Sensor device according to claim 2, **characterised in that** the activation apparatus is a component of the positioning aid apparatus (5) or is coupled thereto.

6. Sensor device according to any of the preceding claims, **characterised in that** the interface is wireless.

7. Sensor device according to any of the preceding claims, **characterised in that** the store for electrical energy is rechargeable, whereby the sensor device has an inductive charging interface for an inductive charging apparatus.

8. Sensor device according to any of the preceding claims, **characterised in that** the motion sensor is an acceleration sensor.

9. Sensor device according to any of the preceding claims, **characterised in that** it has a hook and loop fastener on the region of the housing wall which comes into contact with the chest.

10. Sensor device according to any of the preceding claims, **characterised in that** the measuring standard consists of fibre-reinforced plastic.

11. Sensor device according to any of the preceding claims, **characterised in that** it comprises an inductive sensor for detecting electric currents flowing through the chest of the patient.

12. Sensor device according to any of the preceding claims, **characterised in that** the fastening means (2) is designed in the form of two flexible adhesive strips which are arranged laterally and opposite one another on the housing body (1) and can be pulled out of the housing body (1) or folded away from the housing body (1) for fastening of the sensor device.

13. Sensor device according to any of the preceding claims, **characterised in that** the adhesive coating (6) is a gel or silicone.

14. Use of the sensor device according to any of the preceding claims for analysis of the motion sequence of a living being or a machine.

## Revendications

1. Appareil capteur pour assister un assistant dans une réanimation cardio-pulmonaire d'un patient affecté par un arrêt cardiovasculaire, comprenant des moyens de fixation (2) pour fixer l'appareil capteur à la poitrine du patient, un capteur de mouvement et une interface pour transmettre et/ou recevoir des données et/ou des commandes,
dans lequel l'appareil capteur présente un corps de boîtier (1) sensiblement plat, étanche à l'eau, dans lequel le capteur de mouvement, un stockage pour l'énergie électrique, un processeur et l'interface sont enfermés, et dans lequel le corps de boîtier (1) présente une face supérieure qui, dans l'utilisation prévue de l'appareil capteur, entre en contact avec une main de l'assistant, et une face inférieure qui, dans l'utilisation prévue de l'appareil capteur, entre en contact avec la poitrine du patient,
**caractérisé en ce que**,
l'appareil capteur présente en outre :
- un dispositif d'aide au positionnement (5) pour un positionnement exact de l'appareil capteur sur la poitrine du patient, dans lequel le dispositif d'aide au positionnement (5) est une règle de mesure qui peut être dépliée ou retirée du corps de boîtier (1) ; et
- un revêtement adhésif (6) au moins sur une zone de surface de la face supérieure du corps de boîtier (1).

2. Appareil capteur selon la revendication 1, **caractérisé en ce qu'**il présente un dispositif d'activation pour une connexion au moins du processeur avec le stockage pour l'énergie électrique.

3. Appareil capteur selon la revendication 1, **caractérisé en ce que** le dispositif d'activation peut être commuté par l'intermédiaire d'un actionnement d'un point de rupture (4) ou retrait d'une patte à partir du boîtier (1).

4. Appareil capteur selon la revendication 2, **caractérisé en ce que** le dispositif d'activation est un interrupteur d'activation sensible à un déplacement ou à une pression, agencé à l'intérieur du boîtier (1).

5. Appareil capteur selon la revendication 2, **caractérisé en ce que** le dispositif d'activation est un composant du dispositif d'aide au positionnement (5) ou est couplé à celui-ci.

6. Appareil capteur selon l'une quelconque des revendications précédentes, **caractérisé en ce que** l'interface est sans fil.

7. Appareil capteur selon l'une quelconque des revendications précédentes, **caractérisé en ce que** le stockage pour l'énergie électrique peut être rechargé, dans lequel l'appareil capteur présente une interface de charge inductive pour un dispositif de charge inductive.

8. Appareil capteur selon l'une quelconque des revendications précédentes, **caractérisé en ce que** le capteur de mouvement est un accéléromètre.

9. Appareil capteur selon l'une quelconque des revendications précédentes, **caractérisé en ce qu'**il présente une fermeture autoagrippante sur la zone de la paroi de boîtier qui est en contact avec la poitrine.

10. Appareil capteur selon l'une quelconque des revendications précédentes, **caractérisé en ce que** la règle de mesure consiste en une matière plastique renforcée par des fibres.

11. Appareil capteur selon l'une quelconque des revendications précédentes, **caractérisé en ce qu'**il comprend un capteur inductif pour détecter les courants électriques qui circulent dans la poitrine du patient.

12. Appareil capteur selon l'une quelconque des revendications précédentes, **caractérisé en ce que** le moyen de fixation (2) est réalisé sous la forme de deux bandes adhésives flexibles qui sont agencées latéralement et l'une en face de l'autre sur le corps de boîtier (1) et qui peuvent être retirées du corps de boîtier (1) ou repliées à l'écart du corps de boîtier (1) pour une fixation de l'appareil capteur.

13. Appareil capteur selon l'une quelconque des revendications précédentes, **caractérisé en ce que** le revêtement adhésif (6) est un gel ou une silicone.

14. Utilisation de l'appareil capteur selon l'une quelconque des revendications précédentes pour une analyse de la séquence de mouvements d'un être vivant ou d'une machine.
